# EUROPÄISCHE PATENTSCHRIFT

(11) **EP 1 362 847 B1**
(45) Veröffentlichungstag und Bekanntmachung des Hinweises auf die Patenterteilung: **06.09.2006**
(21) Anmeldenummer: 03010096.0
(22) Anmeldetag: 05.05.2003
(51) Int. Cl.: C07C 263/10, C07C 265/14

(54) **Verfahren zur Herstellung von Isocyanaten in der Gasphase**
Process for the preparation of isocyanates in the gas phase
Procédé de préparation d'isocyanates en phase gazeuze

(30) Priorität: 17.05.2002 DE 10222023
(43) Veröffentlichungstag der Anmeldung: 19.11.2003
(73) Patentinhaber: Bayer MaterialScience AG, 51368 Leverkusen (DE)
(72) Erfinder: Jenne, Marc, Dr., 51065 Köln (DE); Herold, Heiko, 41470 Neuss (DE); Friedrich, Martin, Dr., 51069 Köln (DE); Stutz, Herbert, Dr., 41541 Dormagen (DE)

(56) Entgegenhaltungen:
- EP-A- 0 570 799
- US-A- 5 391 683

## Beschreibung

Die Erfindung betrifft ein Verfahren zur Herstellung von Isocyanaten in der Gasphase, bei dem durch verbesserte Reaktionsführung in Rohrreaktoren durch strömungstechnische Maßnahmen wie der Vergleichmäßigung und Zentrierung der Eduktströme zeitliche Schwankungen der Temperatur und eine Asymmetrie in der Temperaturverteilung vermieden werden. In der Folge wird die Bildung polymerer Nebenprodukte vermieden, die zu Anbackungen im Reaktor und Verkürzung der Standzeit der Reaktoren führen.

In der EP-A 0 289 840 wird ein Verfahren zur Herstellung von (cyclo)aliphatischen Diisocyanaten durch Phosgenierung der entsprechenden, dampfförmigen (cyclo)-aliphatischen Diamine bei 200°C bis 600°C beschrieben. Phosgen wird in stöchiometrischem Überschuß zugeführt. Die überhitzten Ströme von dampfförmigem (cyclo)aliphatischem Diamin bzw. (cyclo)aliphatischem Diamin-Inertgas-Gemisch einerseits und von Phosgen andererseits werden kontinuierlich in einen zylindrischen Reaktionsraum geleitet, dort miteinander vermischt und zur Reaktion gebracht. Die exotherme Phosgenierreaktion wird unter Aufrechthaltung einer turbulenten Strömung durchgeführt.

Gasförmige Edukte werden häufig in Rohrreaktoren reaktionstechnisch umgesetzt. Beim Strahlmischerprinzip (Chemie-Ing.-Techn. 44 (1972) S. 1055, Abb. 10) werden dem Rohrreaktor zwei Eduktströme A und B zugeführt, wobei der Eduktstrom A über eine Zentraldüse und der Eduktstrom B über einen Ringraum zwischen Zentraldüse und Rohrreaktorwand zugeführt werden. Die Strömungsgeschwindigkeit des Eduktstroms A ist groß gegenüber der Strömungsgeschwindigkeit des Eduktstroms B. Hierdurch kommt es im Rohrreaktor zu einer intensiven Vermischung der Reaktionspartner und in der Folge zu deren Reaktion. Diese Art der Reaktionsführung hat technische Bedeutung bei der Herstellung von aromatischen Diisocyanaten durch Phosgenierung von aromatischen Diaminen in der Gasphase erlangt (z.B. EP-A 0 570 799).

Die bekannten Verfahren zeigen jedoch starke Temperaturschwankungen von bis zu 50°C bei der Reaktionsführung. Des Weiteren ist eine Asymmetrie der Temperaturverteilung von bis zu 100°C in Umfangsrichtung des zylindrischen Reaktionsraumes bzw. des Rohrreaktors z.B. mit Thermoelementen messbar.

Folge der Temperaturschwankungen und Asymmetrien in der Temperaturverteilung ist die Bildung polymerer Nebenprodukte, die zu Anbackungen und Verstopfungen im Reaktor und damit zur Verkürzung der Standzeit der Reaktoren führen.

Die Aufgabe der Erfindung ist es daher, ein Verfahren zur Herstellung von (cyclo)aliphatischen und aromatischen Diisocyanaten durch Phosgenierung entsprechender (cyclo)aliphatischer und aromatischer Diamine in der Gasphase bei hohen Temperaturen zur Verfügung zu stellen, bei dem die Temperaturschwankungen in der Reaktionszone und die Asymmetrien in der Temperaturverteilung weitestgehend vermieden werden.

Es wurde nun überraschenderweise gefunden, dass eine Vergleichmäßigung des über den Ringraum des Rohrreaktors zugeführten Eduktstromes B und eine möglichst zentrale Zuführung beider Eduktströme A und B in den Rohrreaktor einen großen positiven Einfluss auf die Stabilität der Reaktionszone und damit auf die Gasphasenreaktion insgesamt hat. Als Folge der stabileren Reaktionsführung nehmen die beobachteten Temperaturschwankungen deutlich ab, die Asymmetrie in der Temperaturverteilung verschwindet praktisch vollständig. Somit können die Nachteile der Verfahren des Standes der Technik deutlich reduziert werden, wenn die Eduktströme den erfindungsgemäßen, nachstehend näher beschriebenen Maßnahmen unterworfen werden.

Gegenstand der Erfindung ist ein Verfahren zur Herstellung von Diisocyanaten und Triisocyanaten der allgemeinen Formel (I)

R(NCO)ₙ (I),

in welcher
- R: für einen (cyclo)aliphatischen oder aromatischen Kohlenwasserstoffrest mit bis zu 15 Kohlenstoffatomen, vorzugsweise 4 bis 13 Kohlenstoffatomen, steht, mit der Maßgabe, dass zwischen zwei NCO-Gruppen mindestens 2 Kohlenstoffatome angeordnet sind und
- n: für die Zahl 2 oder 3 steht,
durch Phosgenierung der entsprechenden Diamine und/oder Triamine der allgemeinen Formel (II) in der Gasphase

R(NH₂)ₙ (II),

in welcher
- R: für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
- n: für die Zahl 2 oder 3 steht,
dadurch gekennzeichnet, dass die Phosgenierung in einem Rohrreaktor durchgeführt wird, der eine Zentraldüse und einen Ringraum zwischen der Zentraldüse und der Wand des Rohrreaktors enthält, wobei die Zentraldüse durch mindestens zwei in dem Ringraum angeordnete Strömungsvergleichmäßiger in dem Rohrreaktor zentriert ist, und wobei die Zentraldüse über ein flexibles Verbindungsrohr mit einem Einlass für einen der Eduktströme verbunden ist und in dem Ringraum der Einlass für einen zweiten Eduktstrom angeordnet ist, und
wobei der die Diamine und / oder Triamine enthaltende Eduktstrom dem Rohrreaktor über die Zentraldüse zugeführt wird, und der Phosgen enthaltende Eduktstrom dem Rohrreaktor über den Ringraum zugeführt wird und die Geschwindigkeit der Strömung im Ringraum mit den Strömungsvergleichmäßigem über dem gesamten Querschnitt des Ringraumes vergleichmäßigt wird.

In einer alternativen Ausführungsform des erfindungsgemäßen Verfahrens werden der die Diamine und / oder Triamine enthaltende Eduktstrom und der Phosgen enthaltende Eduktstrom vertauscht, so dass der die Diamine und / oder Triamine enthaltende Eduktstrom dem Rohrreaktor über den Ringraum und der Phosgen enthaltende Eduktstrom dem Rohrreaktor über die Zentraldüse zugeführt wird.

Vorzugsweise wird erfindungsgemäß der zweite Strömungsvergleichmäßiger oder, wenn es sich um mehr als zwei Strömungsvergleichmäßiger handelt, der letzte Strömungsvergleichmäßiger vor Eintritt des Eduktstromes B in den Rohrreaktor als Kombination ausgeführt. Diese Kombination besteht aus einem Strömungsvergleichmäßiger und einem Strömungsgleichrichter.

Aufgabe der Strömungsvergleichmäßiger ist die Vergleichmäßigung des in den Ringraum dosierten Eduktstromes in der Strömungsgeschwindigkeit über den gesamten Querschnitt des Ringraums. Als Strömungsvergleichmäßiger sind u.a. Lochbleche, Siebe, Sintermetall, Fritten oder Schüttungen bekannt. Bevorzugt werden Lochbleche eingesetzt.

Aufgabe des Strömungsgleichrichters ist die axiale Ausrichtung der Strömung, d.h. schräge Anströmung und Drall werden verhindert. Als Strömungsgleichrichter sind u.a. Wabenstrukturen und Rohrstrukturen bekannt.

Als flexible Verbindungsrohre sind Schläuche und bevorzugt Kompensatoren (wellenförmige Schläuche beispielsweise bestehend aus Edelstahl) geeignet.

Durch das erfindungsgemäße Verfahren gelingt es, eine Abweichung der lokalen Strömungsgeschwindigkeit von der über den gesamten Querschnitt gemittelten Strömungsgeschwindigkeit des über den Ringraum dosierten Eduktstroms von höchstens ± 10 %, vorzugsweise ± 2 % einzustellen.

Im erfindungsgemäßen Verfahren werden Diisocyanate und/oder Triisocyanate aus den entsprechenden Diaminen und/oder Triaminen hergestellt.

Vorzugsweise werden im erfindungsgemäßen Verfahren Diisocyanate durch Phosgenierung der entsprechenden Diamine hergestellt.

Als Triisocyanat der Formel (I) wird im erfindungsgemäßen Verfahren vorzugsweise 1,8-Diisocyanato-4-(isocyanatomethyl)octan, Triisocyanatononan (TIN), hergestellt.

Typische Beispiele geeigneter aliphatischer Diamine sind z.B. in EP-A 0 289 840, typische Beispiele geeigneter aliphatischer Triamine sind z.B. in EP-A 749 958 genannt. Diese Diamine sind geeignet für die Herstellung der entsprechenden Diisocyanate oder Triisocyanate nach dem erfindungsgemäßen Verfahren.

Bevorzugt werden Isophorondiamin (IPDA), Hexamethylendiamin (HDA) und Bis(p-aminocyclohexyl)methan.

Typische Beispiele geeigneter aromatischer Diamine sind die reinen Isomeren oder die Isomerengemische des Diaminobenzols, des Diaminotoluols, des Diaminodimethylbenzols, des Diaminonaphthalins sowie des Diaminodiphenylmethans, bevorzugt sind 2,4/2,6-Toluylendiamin-Gemische der Isomerenverhältnisse 80/20 und 65/35 oder das reine 2,4-Toluylendiamin-Isomere.

Als Triamin wird vorzugsweise 1,8-Diamino-4-(aminomethyl)octan, Triaminononan eingesetzt.

Die Ausgangsamine der Formel (II) werden dem Reaktor gasförmig zugeführt und gegebenenfalls vor der Durchführung des erfindungsgemäßen Verfahrens verdampft und bevorzugt auf 200°C bis 600°C, besonders bevorzugt 250°C bis 450°C erhitzt und gegebenenfalls verdünnt mit einem Inertgas wie N₂, Ne, He, Ar oder mit den Dämpfen eines inerten Lösungsmittels dem Reaktor zugeführt. Das Phosgen wird dem Rohrreaktor in stöchiometrischem Überschuss und bei 200°C bis 600°C zugeführt. Bei Einsatz der aliphatischen Diamine beträgt der molare Phosgenüberschuss bezogen auf eine Aminogruppe bevorzugt zwischen 25 % und 250 %, bei Einsatz der aliphatischen Triamine bevorzugt zwischen 50 % und 350 %. Beim Einsatz aromatischer Diamine beträgt der molare Phosgenüberschuss bezogen auf eine Aminogruppe bevorzugt zwischen 150 % und 300 %.

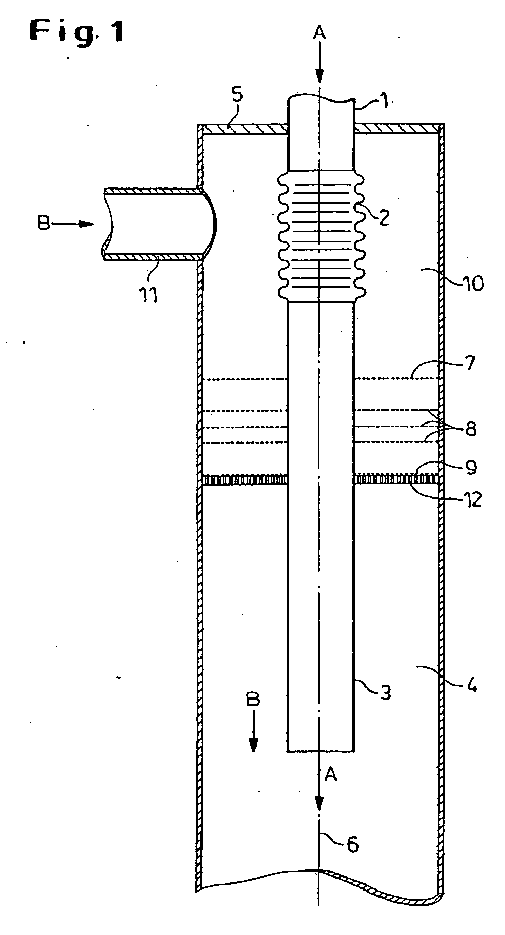

Im Folgenden wird die Erfindung beispielhaft anhand der Fig. 1 erläutert. Der Eduktstrom A (Diamin und / oder Triamin) strömt über den Einlass 1, das flexible Verbindungsrohr 2 und die Zentraldüse 3 in den Rohrreaktor 4. Der Einlass 1 für den Eduktstrom A ist mit dem Deckel 5 des Rohrreaktors 4 starr verbunden. Die Zentraldüse 3 ist über das flexible Verbindungsrohr 2 mit dem Einlass 1 für den Eduktstrom A beweglich verbunden.

Die Zentraldüse 3 wird von mehreren Strömungsvergleichmäßigern 7, 8 und 9 fest in Position gehalten und ist auf die Rotationsachse 6 des Rohrreaktors 4 zentriert. Dazu sind wenigstens zwei Strömungsvergleichmäßiger 7 und 9 notwendig, alle weiteren Strömungsvergleichmäßiger 8 sind optional. Die Strömungsvergleichmäßiger 7, 8 und 9 sind in den Ringraum 10 des Rohrreaktors fest eingebaut und erstrecken sich über den gesamten durchströmten Querschnitt des Ringraums.

Der Eduktstrom B (Phosgen) strömt durch den Einlass 11 in den Ringraum 10 des Rohrreaktors 4. Die Geschwindigkeit des Eduktstroms B wird während der Durchströmung der Strömungsvergleichmäßiger 7, 8 und 9 vergleichmäßigt, so dass eine über den gesamten durchströmten Querschnitt des Ringraums 10 vergleichmäßigte Strömung resultiert. In Strömungsrichtung direkt hinter dem letzten Strömungsvergleichmäßiger 9 ist ein Strömungsgleichrichter 12 angeordnet.

Durch ihn erfährt der Eduktstrom B zusätzlich eine axiale Ausrichtung der Strömungsgeschwindigkeit in Richtung der Rotationsachse 6.

### Beispiel

Einem Rohrreaktor gemäß Fig. 1 werden als Eduktstrom A ein Hexamethylendiamin (HDA)-Inertgas-Gemisch und als Eduktstrom B Phosgen kontinuierlich zugeleitet. Die Temperaturen der beiden Eduktströme betragen 300 Grad Celsius.

Der Versuch wird zum einen erfindungsgemäß durchgeführt mit einem Rohrreaktor gemäß Figur 1, der zwei Strömungsvergleichmäßiger 7 und 9 aufweist. Die Strömungsvergleichmäßiger 8 und der Strömungsgleichrichter 12 sind in dem im erfindungsgemäßen Versuch eingesetzten Rohrreaktor 4 nicht vorhanden.

Zum anderen wird der Versuch als Vergleichsbeispiel durchgeführt mit einem Rohrreaktor, der anstelle der Strömungsvergleichmäßiger 7 und 9 eine Packung enthält, welche die Vergleichmäßigung und Zentrierung der Eduktströme gewährleisten soll. Das flexible Verbindungsrohr 2 wurde ersetzt durch eine starre Verbindung der Zentraldüse 3 mit dem Einlass 1 in den Rohrreaktor 4.

An drei stromabwärts der Zentraldüse 3 gelegenen Messebenen wird die Temperatur auf der zylindrischen Außenwand des Rohrreaktors 4 mit Hilfe von Thermoelementen gemessen. Je Meßebene sind bei einer Kreisteilung von 120° drei Thermoelemente auf der Außenwand angebracht.

Bei dem als Vergleichsbeispiel durchgeführten Verfahren treten an den Messstellen zeitliche Temperaturschwankungen von bis zu 50°C auf. Des weiteren zeigt sich eine Asymmetrie der Temperaturverteilung in Umfangsrichtung von bis zu 100°C. Bei dem erfindungsgemäß durchgeführten Verfahren reduzieren sich die zeitlichen Temperaturschwankungen deutlich auf nunmehr ca. 10°C, die Asymmetrien verschwinden vollständig. Damit wird die Bildung von polymeren Nebenprodukten, die sich an der Wand des Rohrreaktors niederschlagen, reduziert. Dies führt zu einer deutlichen Verlängerung der Standzeit des Reaktors.

## Patentansprüche

1. Verfahren zur Herstellung von Diisocyanaten und Triisocyanaten der allgemeinen Formel (I)
R(NCO)ₙ (I),
in welcher
R für einen (cyclo)aliphatischen oder aromatischen Kohlenwasserstoffrest mit bis zu 15 Kohlenstoffatomen, vorzugsweise 4 bis 13 Kohlenstoffatomen, steht, mit der Maßgabe, dass zwischen zwei NCO-Gruppen mindestens 2 Kohlenstoffatome angeordnet sind und
n für die Zahl 2 oder 3 steht,
durch Phosgenierung der entsprechenden Diamine und/oder Triamine der allgemeinen Formel (II) in der Gasphase
R(NH₂)ₙ (II),
in welcher
R für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
n für die Zahl 2 oder 3 steht,
**dadurch gekennzeichnet, dass** die Phosgenierung in einem Rohrreaktor (4) durchgeführt wird, der eine Zentraldüse (3) und einen Ringraum (10) zwischen der Zentraldüse (3) und der Wand des Rohrreaktors (4) enthält, wobei die Zentraldüse (3) durch mindestens zwei in dem Ringraum (10) angeordnete Strömungsverleichmäßiger (7, 8, 9) in dem Rohrreaktor (4) zentriert ist, und wobei die Zentraldüse (3) über ein flexibles Verbindungsrohr (2) mit einem Einlass (1) für einen der Eduktströme verbunden ist und in dem Ringraum (10) der Einlass (11) für einen zweiten Eduktstrom angeordnet ist, und
wobei der die Diamine und / oder Triamine enthaltende Eduktstrom dem Rohrreaktor (4) über die Zentraldüse (3) zugeführt wird, und der Phosgen enthaltende Eduktstrom dem Rohrreaktor (4) über den Ringraum (10) zugeführt wird und die Geschwindigkeit der Strömung im Ringraum (10) mit den Strömungsvergleichmäßigern (7, 8, 9) über den gesamten Querschnitt des Ringraumes (10) vergleichmäßigt wird.

2. Verfahren nach Anspruch 1, bei dem der die Diamine und / oder Triamine enthaltende Eduktstrom und der Phosgen enthaltende Eduktstrom vertauscht sind, so dass der die Diamine und / oder Triamine enthaltende Eduktstrom dem Rohrreaktor (4) über den Ringraum (10) und der Phosgen enthaltende Eduktstrom dem Rohrreaktor (4) über die Zentraldüse (3) zugeführt wird.

3. Verfahren nach Anspruch 1 oder 2, **dadurch gekennzeichnet, dass** Diisocyanate durch Phosgenierung der entsprechenden Diamine hergestellt werden.

4. Verfahren nach Anspruch 1, **dadurch gekennzeichnet, dass** dem Rohrreaktor (4) dampfförmige (cyclo)aliphatische und / oder aromatische Diamine bei 200°C bis 600°C über die Zentraldüse (3) zugeführt werden und Phosgen dem Rohrreaktor (4) in stöchiometrischem Überschuss bei 200°C bis 600°C über den Ringraum (10) zugeführt wird.

5. Verfahren nach einem der Ansprüche 1 bis 4, **dadurch gekennzeichnet, dass** Isophorondiamin (IPDA) oder Hexamethylendiamin (HDA) oder Bis(p-aminocyclohexyl)methan als Diamine eingesetzt werden.

6. Verfahren nach einem der Ansprüche 1 bis 4, **dadurch gekennzeichnet, dass** 2,4/2,6-Toluylendiamin-Gemische der Isomerenverhältnisse 80/20 und 65/35 oder das reine 2,4-Toluylendiamin-Isomere als Diamine eingesetzt werden.

7. Verfahren nach Anspruch 1 oder 2, **dadurch gekennzeichnet, dass** Triisocyanatononan hergestellt wird.

## Claims

1. Process for preparing diisocyanates and triisocyanates of the general formula (I)
R(NCO)ₙ (I)
where
R is a (cyclo)aliphatic or aromatic hydrocarbon radical having up to 15 carbon atoms, preferably from 4 to 13 carbon atoms, with the proviso that at least two carbon atoms are present between two NCO groups and
n is 2 or 3,
by phosgenation of the corresponding diamines and/or triamines of the general formula (II) in the gas phase
R(NH₂)ₙ (II)
where
R is a (cyclo)aliphatic or aromatic hydrocarbon radical having up to 15, preferably from 4 to 13, carbon atoms, with the proviso that at least two carbon atoms are present between two amino groups and
n is 2 or 3,
**characterized in that** the phosgenation is carried out in a tube reactor (4) having a central nozzle (3) and an annular space (10) between the central nozzle (3) and the wall of the tube reactor (4), with the central nozzle (3) being centred in the tube reactor (4) by means of at least two flow equalizers (7, 8, 9) located in the annular space (10) and the central nozzle (3) being connected via a flexible connecting tube (2) to an inlet (1) for one of the feed streams and the inlet (11) for a second feed stream being located in the annular space (10) and
the feed stream containing the diamines and/or triamines being fed into the tube reactor (4) via the central nozzle (3) and the phosgene-containing feed stream being fed to the tube reactor (4) via the annular space (10) and the velocity of the flow in the annular space (10) being made uniform over the entire cross section of the annular space (10) by means of the flow equalizers (7, 8, 9).

2. Process according to Claim 1, in which the feed stream containing the diamines and/or triamines and the phosgene-containing feed stream are interchanged so that the feed stream containing the diamines and/or triamines is fed into the tube reactor (4) via the annular space (10) and the phosgene-containing feed stream is fed into the tube reactor (4) via the central nozzle (3).

3. Process according to Claim 1 or 2, **characterized in that** diisocyanates are prepared by phosgenation of the corresponding diamines.

4. Process according to Claim 3, **characterized in that** gaseous (cyclo)aliphatic and/or aromatic diamines at from 200°C to 600°C are fed into the tube reactor (4) via the central nozzle (3) and phosgene is fed into the tube reactor (4) in a stoichiometric excess at from 200°C to 600°C via the annular space (10).

5. Process according to any of Claims 1 to 4, **characterized in that** isophoronediamine (IPDA) or hexamethylenediamine (HAD) or bis(p-aminocyciohexyl)methane are used as diamines.

6. Process according to any of Claims 1 to 4, **characterized in that** 2,4-/2,6-toluenediamine mixtures having isomer ratios of 80/20 and 65/35 or the pure 2,4-toluenediamine isomer are used as diamines.

7. Process according to any of Claims 1 or 2, **characterized in that** triisocyanatononane is prepared.

## Revendications

1. Procédé de préparation de diisocyanates et de triisocyanates de la formule générale (I) :
R(NCO)ₙ (I)
dans laquelle
R représente un reste hydrocarbure (cyclo)aliphatique ou aromatique avec jusqu'à 15 atomes de carbone, de préférence 4 à 13 atomes de carbone, avec la condition qu'entre deux groupes NCO, au moins 2 atomes de carbone sont disposés, et
n représente le nombre 2 ou 3,
par phosgénation des diamines et/ou triamines correspondantes, de la formule générale (II) en phase gazeuse
R(NH₂)ₙ (II)
dans laquelle
R représente un reste hydrocarbure (cyclo)aliphatique ou aromatique avec jusqu'à 15 atomes de carbone, de préférence 4 à 13 atomes de carbone, avec la condition qu'entre deux groupes amino, au moins 2 atomes de carbone sont disposés, et
n représente le nombre 2 ou 3,
**caractérisé en ce que** la phosgénation est réalisée dans un réacteur tubulaire (4), qui présente une buse centrale (3) et un espace annulaire (10) entre la buse centrale (3) et la paroi du réacteur tubulaire (4), où la buse centrale (3) est centrée dans le réacteur tubulaire (4) par au moins deux égaliseurs d'écoulement (7, 8, 9) disposés dans l'espace annulaire (10), et où la buse centrale (3) est reliée par un tube de connexion flexible (2) à une entrée (1) pour un des courants de réactif et l'entrée (11) pour un deuxième courant de réactif est disposée dans l'espace annulaire (10), et où le courant de réactif contenant la diamine et/ou triamine est alimenté au réacteur tubulaire (4) par la buse centrale (3), et le courant de réactif contenant le phosgène est alimenté au réacteur tubulaire (4) par l'espace annulaire (10), et la vitesse d'écoulement dans l'espace annulaire (10) est égalisée sur la section transversale de l'espace annulaire (10), par les égaliseurs d'écoulement (7, 8, 9).

2. Procédé suivant la revendication 1, dans lequel le courant de produit contenant la diamine et/ou la triamine et le courant de produit contenant le phosgène sont échangés, de sorte que le courant de produit contenant la diamine et/ou la triamine est alimenté au réacteur tubulaire (4) par l'espace annulaire (10) et le courant de produit contenant le phosgène est alimenté au réacteur tubulaire (4) par la buse centrale (3).

3. Procédé suivant la revendication 1 ou 2, **caractérisé en ce que** les diisocyanates sont préparés par phosgénation des diamines correspondantes.

4. Procédé suivant la revendication 1, **caractérisé en ce que** le réacteur tubulaire (4) est alimenté de la diamine (cyclo)aliphatique et/ou aromatique sous forme gazeuse de 200°C à 600°C par la buse centrale (3) et le phosgène est alimenté au réacteur tubulaire (4) en excès stoeichiométrique de 200°C à 600°C, par l'espace annulaire (10).

5. Procédé suivant l'une quelconque des revendications 1 à 4, **caractérisé en ce que** l'on met en oeuvre l'isophoronediamine (IPDA) ou l'hexaméthylène-diamine (HDA) ou le bis(p-aminocyclohexyl)méthane comme diamine.

6. Procédé suivant l'une quelconque des revendications 1 à 4, **caractérisé en ce que** l'on met en oeuvre le mélange 2,4/2,6-tolyulènediamine des rapports isomériques 80/20 et 65/35 ou l'isomère 2,4-toluylènediamine pur comme diamine.

7. Procédé suivant la revendication 1 ou 2, **caractérisé en ce que** l'on prépare le triisocyanatononane.
